# FASCICULE DE BREVET EUROPEEN

(11) **EP 3 593 764 B1**
(45) Date de publication et mention de la délivrance du brevet: **23.06.2021**
(21) Numéro de dépôt: 19186101.2
(22) Date de dépôt: 12.07.2019
(51) Int. Cl.: A61F 2/30, A61F 2/40

(54) **IMPLANT DE GLÈNE DE PROTHÈSE D'ÉPAULE ET PROCÉDÉ POUR LA FABRICATION DE CET IMPLANT**
GELENKPFANNEN-IMPLANTAT EINER SCHULTERPROTHESE, UND VERFAHREN ZUR HERSTELLUNG EINES SOLCHEN IMPLANTATS
GLENOID BONE IMPLANT FOR SHOULDER PROSTHESIS AND METHOD FOR MANUFACTURING SAID IMPLANT

(30) Priorité: 13.07.2018 FR 1856487
(43) Date de publication de la demande: 15.01.2020
(73) Titulaire: FX Solutions, 01000 Bourg en Bresse (FR)
(72) Inventeur: ASWAD, Richard, 13009 Marseille (FR); BARGOUIN, Kevin, 44400 Rezé (FR); CERMEÑO, Roberto, 40160 Torrecaballeros (Segovia) (ES); CHAOUAT, Gilles, 77330 Ozoir la Ferriere (FR); LASCAR, Tristan, 98000 Monaco (MC); MORA, José María, 08006 Barcelona (ES); NOURISSAT, Geoffroy, 94300 Vincennes (FR)
(74) Mandataire: LLR

(56) Documents cités:
- EP-A1- 2 465 549
- WO-A1-2011/138646
- US-A1- 2005 112 397
- US-A1- 2014 277 517

## Description

La présente invention concerne un implant de glène de prothèse d'épaule et un procédé pour la fabrication de cet implant.

Comme cela est bien connu, un implant prothétique de glène est destiné à être placé sur l'omoplate de l'articulation de l'épaule à restaurer afin de former une surface articulaire, cette surface articulaire étant apte à coopérer avec une surface articulaire conjuguée formée par l'implant prothétique huméral.

Un tel implant de glène doit reproduire dans toute la mesure du possible les caractéristiques de la partie osseuse qu'il remplace ; or, les implants de glène existants ne sont pas parfaits de ce point de vue. De plus, la mise en place d'un tel implant doit préserver autant que possible le capital osseux du patient, ce que ne permettent souvent pas parfaitement les implants de glène existants.

L'objectif principal de l'invention est de remédier à ces inconvénients tout en fournissant un implant présentant une structure résistante, apte à résister aux efforts répétés générés par les mouvements du patient.

Les documents WO 2011/138646 ou US 2005/112397 montrent des structures d'implants ne permettant pas d'atteindre cet objectif.

L'implant concerné comprend, de manière connue en soi,
- une embase d'ancrage destinée à être ancrée à l'os de l'omoplate, et un plateau de glissement en un matériau thermoplastique favorisant le glissement, l'embase d'ancrage formant une partie de support pour recevoir ledit plateau de glissement ;
- la partie de support présente une première paroi destinée à venir en contact avec l'os et une deuxième paroi coextensive et séparée de la première paroi, mais solidaire de celle-ci, cette deuxième paroi présentant une pluralité d'ouvertures aménagées au travers d'elle,
- le matériau dudit plateau de glissement s'étend au travers des ouvertures que présente la deuxième paroi, jusque dans l'espace délimité entre ladite première paroi et ladite deuxième paroi, et est répandu au-delà du périmètre de chaque ouverture, de sorte que ce matériau remplisse au moins partiellement ledit espace.

Selon l'invention,
les deux parois présentent ladite forme arrondie, de telle sorte que ladite première paroi forme une surface concave son côté tourné vers ladite deuxième paroi, et que cette deuxième paroi forme une surface convexe son côté tourné vers ladite première paroi ;
les formes arrondies des deux parois sont définies par des rayons proches ou identiques d'une paroi à l'autre, de telle sorte que ledit espace entre les deux parois ait une épaisseur sensiblement constante.

Le procédé selon l'invention comprend les étapes consistant à :
a) aménager l'embase d'ancrage que comprend l'implant sous la forme d'une première paroi et d'une deuxième paroi qui sont séparées l'une de l'autre et coextensives l'une par rapport à l'autre de façon à délimiter un espace entre ces parois, ladite deuxième paroi se trouvant sur le côté de cette partie de support destinée à recevoir ce plateau de glissement et présentant une pluralité d'ouvertures au travers d'elle, débouchant dans ledit espace ;
b) placer l'embase d'ancrage dans un moule ;
c) porter le matériau thermoplastique destiné à constituer le plateau de glissement à une température de malléabilité ;
d) à l'intérieur du moule, presser ce matériau contre ladite deuxième paroi de façon à faire fluer ce matériau au travers des ouvertures de ladite deuxième paroi jusque dans ledit espace et à répandre ce matériau dans cet espace au-delà du périmètre de chaque ouverture ;
e) laisser refroidir le matériau thermoplastique de façon à constituer le plateau de glissement ;
f) extraire l'implant obtenu hors du moule.

L'invention consiste ainsi à prévoir une embase d'ancrage ayant non pas une seule paroi épaisse mais en deux parois coextensives et séparées l'une de l'autre de façon à délimiter un espace entre elles. La deuxième paroi présente une pluralité d'ouvertures au travers desquelles le matériau thermoplastique destiné à former le plateau de glissement est destiné à s'étendre, ce matériau étant répandu dans cet espace et venant au moins partiellement remplir ce dernier. Une fois le matériau thermoplastique refroidi, une parfaite liaison du plateau à l'embase de réception est ainsi assurée.

Ladite forme convexe est favorable à la prise d'appui de l'implant contre la surface osseuse correspondante. Le fait que les deux parois présentent ladite forme arrondie permet que ladite première paroi forme une surface concave son côté tourné vers ladite deuxième paroi, et que cette deuxième paroi forme une surface convexe son côté tourné vers ladite première paroi ; ladite deuxième paroi est ainsi plus ou moins logée dans la concavité formée par ladite première paroi, ce qui est favorable à la cohésion des deux parois. En outre, ledit espace entre les deux parois a une épaisseur sensiblement constante, qui est favorable à la bonne répartition du matériau thermoplastique destiné à constituer le plateau de glissement dans ledit espace.

Le matériau thermoplastique peut notamment être du polyéthylène à ultra haut poids moléculaire, du PEEK (Polyétheréthercétone), du PEKK (Polyéthercétonecétone).

De préférence, au moins une des première et deuxième parois présente une épaisseur allant de 0,2 à 0,6 mm.

Il est ainsi possible d'obtenir une partie de support du plateau de glissement ayant, de par l'épaisseur réduite des parois, une légère souplesse permettant une bonne diffusion à l'os des contraintes exercées sur l'omoplate par les mouvements du patient, et ayant de plus une épaisseur globale réduite permettant de préserver au maximum le capital osseux du patient. Cette épaisseur réduite permet de plus de ne pas tirer sur les parties molles de l'articulation.

De préférence, chacune des deux parois a une épaisseur allant de 0,2 à 0,6 mm, et l'espace entre les deux parois a une épaisseur allant de 0,2 à 0,8 mm, de telle sorte que l'épaisseur globale de ladite partie de support soit de l'ordre de 0,6 à 2 mm.

Les résultats de relative souplesse de l'embase d'ancrage et de préservation du capital osseux sont, de cette façon, particulièrement obtenus.

Les deux parois précitées pourraient être réalisées séparément puis être placées dans leur position de co-extension et être ensuite assemblées l'une à l'autre ; selon une forme de réalisation préférée de l'invention, toutefois, la première et la deuxième parois forment corps l'une avec l'autre, étant reliées l'une à l'autre par des ponts de matière d'un seul tenant avec elles, ces ponts reliant la première paroi aux parties de ladite deuxième paroi situées entre les ouvertures que présente cette deuxième paroi.

L'embase d'ancrage ainsi constituée a une structure présentant une parfaite cohésion et peut être obtenu de façon relativement simple et rapide par un procédé d'impression en trois dimensions.

L'implant comprend de préférence des moyens d'ancrage osseux sous la forme :
- d'un plot central solidaire de ladite première paroi, et/ou
- d'au moins un plot non central solidaire de ladite première paroi, et/ou
- d'au moins un trou à vis.

Selon une forme de réalisation préférée de ces moyens d'ancrage osseux, l'implant comprend :
- un plot central,
- sur un premier côté de l'implant, deux plots latéraux, et
- sur un deuxième côté opposé au premier côté par rapport au plot central, un trou à vis.

Le plot central et la vis permettent de réaliser un ancrage profond de l'implant à l'os ; les plots latéraux et la vis permettent d'assurer la stabilité de l'implant à l'égard d'un risque de rotation autour du plot central.

Le plot central peut notamment être tubulaire et présenter un taraudage distal permettant, si nécessaire, le montage d'une pièce de prolongement, et/ou un taraudage proximal permettant le montage d'un instrument de retrait de l'implant par rapport à l'os.

Les moyens d'ancrage osseux peuvent également être sous la forme d'un plot central et de deux trous à vis de part et d'autre de ce plot central.

Les moyens d'ancrage osseux peuvent aussi être sous la forme d'un plot central et d'un ou plusieurs plots latéraux de part et d'autre de ce plot central ; si ces plots latéraux sont aplatis et qu'ils présentent donc une largeur, un premier plot latéral peut avoir sa largeur disposée parallèlement à une direction longitudinale de la partie de support, ou disposée perpendiculairement à cette direction longitudinale ; un deuxième plot latéral situé du côté opposé audit premier plot latéral peut avoir sa largeur disposée perpendiculairement à cette direction longitudinale.

L'invention sera bien comprise, et d'autres caractéristiques et avantages de celle-ci apparaîtront, en référence au dessin schématique annexé, représentant, à titre d'exemple non limitatif, une forme de réalisation préférée de l'implant concerné.
La figure 1 est une vue en perspective, selon un premier angle, d'une embase d'ancrage que comprend l'implant ;
la figure 2 est une vue de cette embase d'ancrage en perspective selon un angle décalé d'environ 90° par rapport à l'angle de vue selon la figure 1 ;
la figure 3 est une vue de l'embase d'ancrage de côté ;
la figure 4 est une vue de l'implant en perspective similaire à la figure 1 ;
la figure 5 est une vue de l'implant en perspective selon un angle décalé d'environ 180° par rapport à l'angle de vue selon la figure 4 ; et
la figure 6 est une vue de l'implant en perspective similaire à la figure 5, avec mise en place d'une vis dans un trou à vis que comprend l'embase d'ancrage.

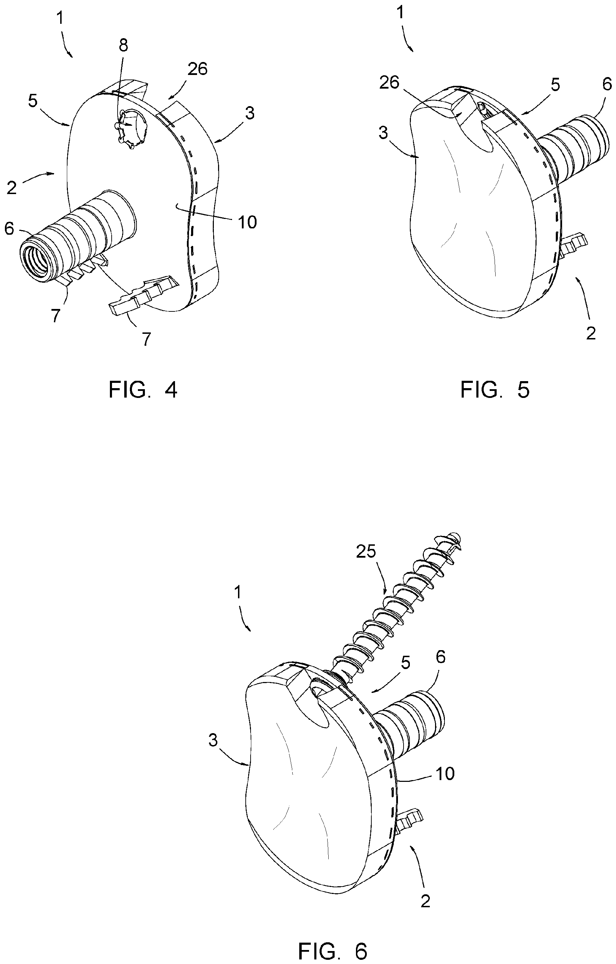

Les figures 4 à 6 représentent un implant de glène 1 de prothèse d'épaule, qui comprend une embase d'ancrage 2 destinée à être ancrée à l'os de l'omoplate, et un plateau de glissement 3 en un matériau thermoplastique favorisant le glissement (tel que UHMWPE, PEEK, PEKK...), en particulier du polyéthylène à haut poids moléculaire.

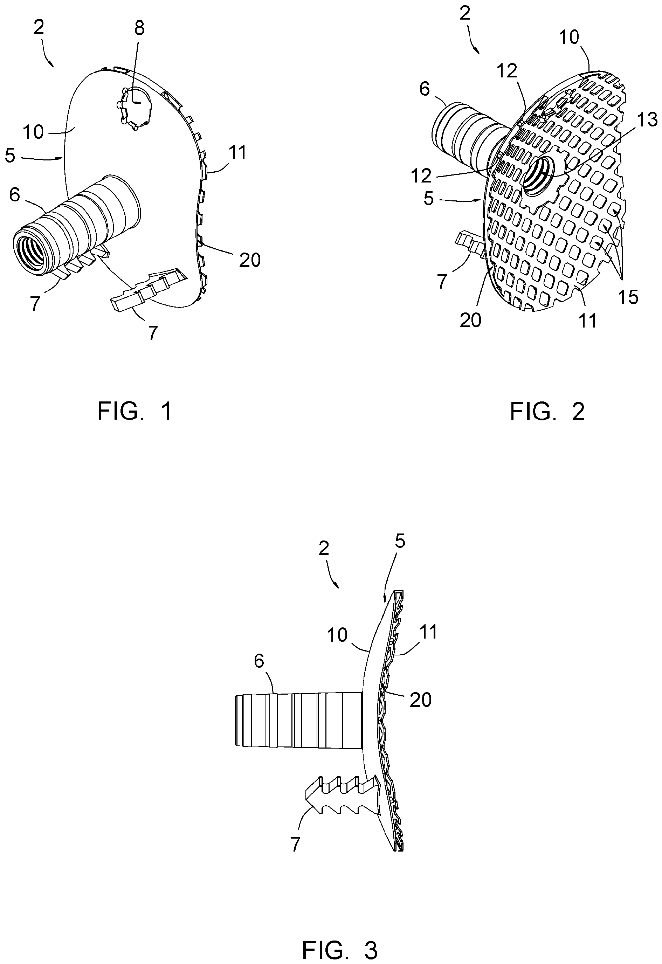

L'embase d'ancrage 2 est visible sur les figures 1 à 3 sans le plateau de glissement 3. Elle forme formant une partie de support 5 pour recevoir ce plateau, un plot central d'ancrage 6, deux plots latéraux d'ancrage 7 sur un premier côté de l'implant du plot 6 et un trou à vis 8 sur un deuxième côté du plot 6 opposé au premier côté.

La partie de support 5 est formée par une première paroi 10 et une deuxième paroi 11 reliée l'une à l'autre par des ponts de matière 12.

La première paroi 10 présente un périmètre en forme d'anneau, est pleine, c'est-à-dire est dépourvue d'ouverture aménagées à travers elle, à l'exception d'une ouverture communiquant avec une cavité 13 formée intérieurement par le plot central 6, lequel est creux, et à l'exception du trou 8, a une épaisseur allant de 0,2 à 0,3 mm, et a une forme doublement arrondie, un premier arrondi étant selon une direction de longueur qui va du côté sur lequel se trouvent les plots 7 au côté sur lequel se trouve le trou 8 et le deuxième arrondi étant selon une direction de largeur perpendiculaire à cette direction de longueur.

Sur sa face convexe destinée à venir en contact avec l'os, convenablement réséqué, d'une omoplate, la paroi 10 présente un revêtement ostéo-inducteur et/ou ostéo-conducteur, possiblement poreux. Un tel revêtement est bien connu en lui-même et n'est donc pas décrit en détail.

La deuxième paroi 11 présente un périmètre identique à celui de la paroi 10, est doublement arrondie selon des rayons de courbure légèrement inférieurs aux rayons des courbures homologues de la paroi 10, a également une épaisseur de l'ordre de 0,2 à 0,6 mm, et présente une pluralité d'ouvertures 15 de forme plus ou moins carrée, disposés selon des alignements perpendiculaires entre eux, disposés en diagonale par rapport aux directions de longueur et de largeur précités.

La paroi 11 est coextensive à la paroi 10 et les ponts de matière 12 relient les parties pleines de cette paroi 11 situées entre les ouvertures 15 à la paroi 10. Ces ponts 12 sont d'un seul tenant avec ces parois 10, 11, l'ensemble de la structure de l'embase 2 est réalisé par un procédé d'impression en trois dimensions, comme décrit plus loin, et permettent, avec les parois 10 et 11, de délimiter entre un espace 20 ayant une épaisseur sensiblement constante, allant de 0,2 à 0,8 mm

Le plot central 6 est creux intérieurement comme déjà mentionné, délimitant une cavité tubulaire qui est taraudée selon des taraudages différents dans sa partie proximale et dans sa partie distale. L'orifice taraudé proximal permet l'ablation de l'implant 1 nécessaire, par perçage du plateau de glissement 3 pour accéder à cet orifice à fin d'insérer un outil permettant d'extraire l'implant de l'os, par exemple lors d'une reprise, c'est-à-dire du changement d'une prothèse préalablement implantée par une nouvelle prothèse. L'orifice taraudé distal permet la mise en place d'une pièce de prolongation du plot 6 si cela est rendu nécessaire par une qualité moindre de l'os.

Extérieurement, le plot 6 présente des rainures permettant de favoriser son accrochage à l'os spongieux.

Les plots 7 présentent chacun une pluralité de dentelures sur deux côtés opposés, qui permettent leur rétention par l'os spongieux lorsque ces plots sont insérés en force dans cet os. Comme cela est visible sur les figures 1 et 4 notamment, ces plots sont aplatis, ce qui leur confère une forme "en sapin", et sont orientées selon des angles par rapport à ladite direction de longueur de l'embase 2, selon des angles par rapport à cette direction, de l'ordre de 120 à 130°.

Le trou 8 est taraudé et présente des encoches radiales sur une portion de sa périphérie, en étant dépourvu de telles encoches radiales sur l'autre portion de sa périphérie ; ce trou est conforme à la publication de demande de brevet N°
au nom de la demanderesse et permet, de par sa structure, d'autoriser l'orientation d'une vis engagée dans ce trou selon certaines directions tout en interdisant l'orientation de la vis dans d'autres directions, ainsi que cela est décrit dans ladite publication de demande de brevet. En l'occurrence, en référence notamment à la figure 1, il apparaît que le trou 8 est dépourvu d'encoches radiales au niveau du quart de sa périphérie se trouvant du côté opposé au plot 6.

Le procédé de fabrication de l'implant 1 comprend les étapes consistant à :
a) aménager l'embase d'ancrage 2 par un procédé dit "d'impression en 3D", de façon à constituer la partie de support 5 et les plots 6, 7 et trou 8 tels que décrits plus haut et tels que visibles sur les figures 1 à 3 ;
b) placer l'embase d'ancrage 2 dans un moule ;
   b1) dans le moule, placer sur l'embase d'ancrage 2, du côté de la paroi 11, une poudre de polyéthylène destiné à constituer le plateau de glissement 3 ;
c) porter cette poudre à une température de malléabilité ;
d) à l'intérieur du moule, presser la poudre contre ladite deuxième paroi de façon à faire fluer le polyéthylène au travers des ouvertures 15 que présente la paroi 11 jusque dans l'espace 20 et à répandre ce polyéthylène dans cet espace 20 au-delà du périmètre de chaque ouverture 15 ;
e) laisser refroidir le polyéthylène de façon à constituer le plateau de glissement 3 ; et
f) extraire l'implant obtenu, qui est tel que visible sur les figures 4 et 5, hors du moule.

Ainsi, le matériau du plateau 3 s'étend au travers des ouvertures 15, jusque dans l'espace 20, et est répandu au-delà du périmètre de chaque ouverture 15, de sorte que ce matériau remplisse au moins partiellement l'espace 20 et qu'il réalise une liaison intime du plateau 3 à l'embase 2.

Le procédé d'impression 3D peut être employé pour réaliser entièrement l'embase d'ancrage 2 ; il peut également n'être utilisé que partiellement, à savoir que l'embase d'ancrage peut comprendre une ébauche obtenue par usinage ou autre technique (forgeage, moulage, etc.), sur laquelle une partie de complément est réalisée par le procédé d'impression 3D

Le matériau thermoplastique destiné à constituer le plateau de glissement peut être sous forme de poudre et le fluage de ce matériau être réalisé par thermocompression ; le plateau de glissement pourrait également être réalisé par injection.

## Revendications

1. Implant (1) de glène de prothèse d'épaule, comprenant une embase d'ancrage (2) destinée à être ancrée à l'os de l'omoplate, et un plateau de glissement (3) en un matériau thermoplastique favorisant le glissement, l'embase d'ancrage (2) formant une partie de support (5) pour recevoir ledit plateau de glissement (3) ;
- la partie de support (5) présente une première paroi (10) destinée à venir en contact avec l'os et une deuxième paroi (11) coextensive et séparée de la première paroi (10), mais solidaire de celle-ci, cette deuxième paroi (11) présentant une pluralité d'ouvertures (15) aménagées au travers d'elle,
- le matériau dudit plateau de glissement (3) s'étend au travers des ouvertures (15) que présente la deuxième paroi (11), jusque dans l'espace (20) délimité entre ladite première paroi (10) et ladite deuxième paroi (11), et est répandu au-delà du périmètre de chaque ouverture (15), de sorte que ce matériau remplisse au moins partiellement ledit espace (20) ;
caractérisé :
les deux parois (10, 11) présentent ladite forme arrondie, de telle sorte que ladite première paroi (10) forme une surface concave son côté tourné vers ladite deuxième paroi (11), et que cette deuxième paroi (11) forme une surface convexe son côté tourné vers ladite première paroi (10) ;
les formes arrondies des deux parois (10, 11) sont définies par des rayons proches ou identiques d'une paroi à l'autre, de telle sorte que ledit espace (20) entre les deux parois (10, 11) ait une épaisseur sensiblement constante.

2. Implant (1) selon la revendication 1, **caractérisé en ce qu'**au moins une des première et deuxième parois (10, 11) présente une épaisseur allant de 0,2 à 0,6 mm.

3. Implant (1) selon la revendication 2, **caractérisé en ce que** chacune des deux parois (10, 11) a une épaisseur allant de 0,2 à 0,6 mm, et **en ce que** l'espace (20) entre les deux parois a une épaisseur allant de 0,2 à 0,8 mm, de telle sorte que l'épaisseur globale de ladite partie de support (5) soit de l'ordre de 0,6 à 2 mm.

4. Implant (1) selon l'une des revendications 1 à 3, **caractérisé en ce que** la première et la deuxième parois (10, 11) forment corps l'une avec l'autre, étant reliées l'une à l'autre par des ponts de matière (12) d'un seul tenant avec elles, ces ponts reliant la première paroi (10) aux parties de ladite deuxième paroi (11) situées entre les ouvertures (15) que présente cette deuxième paroi (11).

5. Implant (1) selon l'une des revendications 1 à 4, **caractérisé en ce qu'**il comprend des moyens d'ancrage osseux sous la forme :
- d'un plot central (6) solidaire de ladite première paroi (10), et/ou
- d'au moins un plot non central (7) solidaire de ladite première paroi (10), et/ou
- d'au moins un trou à vis (8).

6. Procédé de fabrication de l'implant (1) selon l'une des revendications 1 à 5, **caractérisé en ce qu'**il comprend les étapes consistant à :
a) aménager l'embase d'ancrage (2) que comprend l'implant (1) sous la forme d'une première paroi (10) et d'une deuxième paroi (11) qui sont séparées l'une de l'autre et coextensives l'une par rapport à l'autre de façon à délimiter un espace (20) entre ces parois, ladite deuxième paroi (11) se trouvant sur le côté de cette partie de support (5) destinée à recevoir le plateau de glissement (3) et présentant une pluralité d'ouvertures (15) au travers d'elle, débouchant dans ledit espace (20) ;
b) placer l'embase d'ancrage (2) dans un moule ;
c) porter le matériau thermoplastique destiné à constituer le plateau de glissement (3) à une température de malléabilité ;
d) à l'intérieur du moule, presser ce matériau contre ladite deuxième paroi (11) de façon à faire fluer ce matériau au travers des ouvertures (15) de ladite deuxième paroi (11) jusque dans ledit espace (20) et à répandre ce matériau dans cet espace (20) au-delà du périmètre de chaque ouverture (15) ;
e) laisser refroidir le matériau thermoplastique de façon à constituer le plateau de glissement (3) ;
f) extraire l'implant (1) obtenu hors du moule.

7. Procédé selon la revendication 6, de fabrication de l'implant (1) selon la revendication 4, **caractérisé en ce qu'**il comprend l'étape consistant à fabriquer l'embase d'ancrage (2) par un procédé d'impression en trois dimensions, de façon que la première et la deuxième parois (10, 11) forment corps l'une avec l'autre, en étant reliées l'une à l'autre par des ponts de matière (12) d'un seul tenant avec elles, ces ponts reliant la première paroi (10) aux parties de ladite deuxième paroi (11) situées entre les ouvertures (15) que présente cette deuxième paroi (11).

## Patentansprüche

1. Schulterprothesen-Glenoid-Implantat (1), aufweisend eine Verankerungsbasis (2), die dazu bestimmt ist, am Knochen des Schulterblatts verankert zu werden, und eine Gleitplatte (3) aus einem thermoplastischen Material, die das Gleiten fördert, wobei die Verankerungsbasis (2) einen Trägerteil (5) zur Aufnahme der Gleitplatte (3) bildet;
- der Trägerteil (5) eine erste Wand (10), die zum Berühren des Knochens bestimmt ist, und eine zweite Wand (11) aufweist, die koextensiv und getrennt von der ersten Wand (10), aber integral mit dieser ist, wobei die zweite Wand (11) eine Mehrzahl von Öffnungen (15) durch sie hindurch aufweist; **dadurch gekennzeichnet, dass**
- das Material der Gleitplatte (3) sich durch die Öffnungen (15) in der zweiten Wand (11) in den zwischen der ersten Wand (10) und der zweiten Wand (11) definierten Raum (20) erstreckt und über den Umfang jeder Öffnung (15) hinaus ausgebreitet wird, so dass dieses Material den Raum (20) zumindest teilweise ausfüllt;
**dadurch gekennzeichnet, dass**
die beiden Wände (10, 11) die abgerundete Form haben, so dass die erste Wand (10) eine konkave Oberfläche mit ihrer der zweiten Wand (11) zugewandten Seite bildet, und die zweite Wand (11) eine konvexe Oberfläche mit ihrer der ersten Wand (10) zugewandten Seite bildet;
die abgerundeten Formen der beiden Wände (10, 11) durch enge oder identische Radien von einer Wand zur anderen entblößt sind, so dass der Raum (20) zwischen den beiden Wänden (10, 11) eine im Wesentlichen konstante Dicke aufweist.

2. Implantat (1) nach Anspruch 1, **dadurch gekennzeichnet, dass** mindestens eine der ersten und zweiten Wände (10, 11) eine Dicke von 0,2 bis 0,6 mm aufweist.

3. Implantat (1) nach Anspruch 2, **dadurch gekennzeichnet, dass** jede der beiden Wände (10, 11) eine Dicke von 0,2 bis 0,6 mm aufweist, und dass der Zwischenraum (20) zwischen den beiden Wänden eine Dicke von 0,2 bis 0,8 mm aufweist, so dass die Gesamtdicke des Trägerteils (5) im Bereich von 0,6 bis 2 mm liegt.

4. Implantat (1) nach einem der Ansprüche 1 bis 3, **dadurch gekennzeichnet, dass** die erste und die zweite Wand (10, 11) einstückig miteinander ausgebildet sind, wobei sie durch einstückig ausgebildete Materialbrücken (12) miteinander verbunden sind, wobei die Brücken die erste Wand (10) mit den zwischen den Öffnungen (15) in dieser zweiten Wand (11) liegenden Teilen der zweiten Wand (11) verbinden.

5. Implantat (1) nach einem der Ansprüche 1 bis 4, **dadurch gekennzeichnet, dass** es Knochenverankerungsmittel aufweist in Form von:
- einem zentralen Ständer (6), der mit der ersten Wand (10) integriert ist, und/oder
- mindestens einem nicht zentralen Ständer (7), der mit der ersten Wand (10) fest verbunden ist, und/oder
- mindestens ein Schraubenloch (8).

6. Verfahren zur Herstellung des Implantats (1) nach einem der Ansprüche 1 bis 5, **dadurch gekennzeichnet, dass** es die Schritte aufweist, bestehend aus:
a) Anordnen der Verankerungsbasis (2), die das Implantat (1) aufweist, in Form einer ersten Wand (10) und einer zweiten Wand (11), die voneinander getrennt sind und zueinander koextensiv sind, um einen Raum (20) zwischen diesen Wänden zu begrenzen, wobei die zweite Wand (11) sich auf der Seite desjenigen Trägerteils (5) befindet, der zur Aufnahme der Gleitplatte (3) bestimmt ist, und eine Vielzahl von Öffnungen (15) durch sie hindurch aufweist, die in den Raum (20) münden;
b) Einlegen der Verankerungsbasis (2) in eine Form;
c) Erwärmen des thermoplastischen Materials zur Bildung der Gleitplatte (3) auf eine Verformbarkeitstemperatur;
d) innerhalb der Form, Pressen dieses Materials gegen die zweite Wand (11), um zu bewirken, dass dieses Material durch die Öffnungen (15) der zweiten Wand (11) in den Raum (20) fließt und dieses Material in diesem Raum (20) über den Umfang jeder Öffnung (15) hinaus verteilt;
e) Abkühlenlassen des thermoplastischen Materials, um die Gleitplatte (3) zu bilden;
f) Entnehmen des entstandenen Implantats (1) aus der Form.

7. Verfahren nach Anspruch 6 zur Herstellung des Implantats (1) nach Anspruch 4, **dadurch gekennzeichnet, dass** es den Schritt der Herstellung der Verankerungsbasis (2) durch ein dreidimensionales Druckverfahren aufweist, so dass die erste und zweite Wand (10, 11) einstückig miteinander sind, wobei sie durch einstückige Materialbrücken (12) miteinander verbunden sind, die die erste Wand (10) mit den Teilen der zweiten Wand (11) verbinden, die sich zwischen den Öffnungen (15) in dieser zweiten Wand (11) befinden.

## Claims

1. A shoulder prosthesis glenoid implant (1), comprising an anchoring base (2) intended to be anchored to the bone of the scapula, and a sliding plate (3) made of a thermoplastic material enhancing the sliding, the anchoring base (2) forming a support part (5) for receiving said sliding plate (3);
- the support part (5) has a first wall (10) intended to contact the bone and a second wall (11) coextensive with, and separate from, the first wall (10), but integral with the latter, this second wall (11) having a plurality of openings (15) formed through it,
- the material of said sliding plate (3) extends through the openings (15) which the second wall (11) has, into the space (20) delimited between said first wall (10) and said second wall (11), and is spread beyond the perimeter of each opening (15), so that this material at least partially fills said space (20);
**characterized in that**:
the two walls (10, 11) have said rounded shape, such that said first wall (10) forms a concave surface on its side facing said second wall (11), and **in that** this second wall (11) forms a convex surface on its side facing said first wall (10);
the rounded shapes of the two walls (10, 11) are defined by similar or identical radii from one wall to the other, so that said space (20) between the two walls (10, 11) has a thickness substantially constant.

2. The implant (1) according to claim 1, **characterized in that** at least one of the first and second walls (10, 11) has a thickness ranging from 0.2 to 0.6mm.

3. The implant (1) according to claim 2, **characterized in that** each of the two walls (10, 11) has a thickness ranging from 0.2 to 0.6mm, and **in that** the space (20) between the two walls has a thickness ranging from 0.2 to 0.8mm, so that the overall thickness of said support part (5) is of the order of 0.6 to 2mm.

4. The implant (1) according to one of claims 1 to 3, **characterized in that** the first and second walls (10, 11) are made integral one with the other, being connected to each other by means of bridges of material (12) integrally formed with them, these bridges connecting the first wall (10) to the parts of said second wall (11) located between the openings (15) that this second wall (11) has.

5. The implant (1) according to one of claims 1 to 4, **characterized in that** it comprises bone anchoring means in the form of:
- a central stud (6) integral with said first wall (10), and/or
- at least one non-central stud (7) integral to said first wall (10), and/or
- at least one hole (8) for a screw.

6. A method of manufacturing the implant (1) according to one of claims 1 to 5, **characterized in that** it comprises the steps of:
a designing the anchoring base (2) which the implant (1) comprises in the form of a first wall (10) and a second wall (11) which are separated from each other and coextensive with respect to each other so as to define a space (20) between these walls, said second wall (11) being on the side of this support part (5) intended to receive the sliding plate (3) and having a plurality of openings (15) formed through it, emerging into said space (20);
b) placing the anchoring base (2) in a mold;
c) bringing the thermoplastic material intended to constitute the sliding plate (3) to a malleability temperature;
d) inside the mold, pressing this material against said second wall (11) so as to cause this material to flow through the openings (15) of said second wall (11) into said space (20) and to spread this material in this space (20) beyond the perimeter of each opening (15);
e) allowing the thermoplastic material to cool so as to constitute the sliding plate (3);
f) extracting the implant (1) obtained from the mold.

7. The method according to claim 6, for manufacturing the implant (1) according to claim 4, **characterized in that** it comprises the step of manufacturing the anchoring base (2) by a three dimension printing process, so that the first and the second walls (10, 11) are integral one with the other, being connected to each other by bridges of material (12) integral with them, these bridges connecting the first wall (10) to the parts of said second wall (11) located between the openings (15) which this second wall (11) has.
